(19) Europäisches Patentamt
European Patent Office
Office européen des brevets

(11) **EP 3 522 838 B1**

(12) **EUROPEAN PATENT SPECIFICATION**

(45) Date of publication and mention
of the grant of the patent:
**13.05.2020 Bulletin 2020/20**

(21) Application number: **17780380.6**

(22) Date of filing: **04.10.2017**

(51) Int Cl.:
***A61F 5/01*** (2006.01)

(86) International application number:
**PCT/EP2017/075205**

(87) International publication number:
**WO 2018/065459 (12.04.2018 Gazette 2018/15)**

(54) **ARMLIFTING SUPPORT DEVICE**

ARMHEBEUNTERSTÜTZUNGSVORRICHTUNG

DISPOSITIF DE SUPPORT DE LEVAGE DE BRAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.10.2016 SE 1651308**

(43) Date of publication of application:
**14.08.2019 Bulletin 2019/33**

(73) Proprietor: **Bioservo Technologies Aktiebolag 164 40 Kista (SE)**

(72) Inventors:
• **INGVAST, Johan**
**184 42 Åkersberga (SE)**
• **WAHLSTEDT, Martin**
**170 73 Solna (SE)**

(74) Representative: **Noréns Patentbyrå AB Box 10198 100 55 Stockholm (SE)**

(56) References cited:
JP-A- 2012 040 111    JP-A- 2012 040 111
US-A- 4 180 870      US-A- 4 180 870
US-A- 4 559 932      US-A- 4 559 932
US-A- 4 862 878      US-A- 4 862 878
US-A1- 2012 184 880   US-A1- 2012 184 880

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention concerns an arm lifting support device. The arm lifting support device could for example help a person with impaired arm function.

The aim of the present invention is to provide a support device for helping persons having an arm impairment. The arm lifting support should be easy to wear and use in order to enhance the life of the user making it possible for the user to perform movements and functions without needing help from a helping person. A further aim is to provide a support device of a slim design.

It is previously known exoskeleton support devices, which rely on hard parts being connected on the outside of a user's body mimicking the bones and their functions in the body. This creates a very bulky support.

For example to be able to apply force to lift the arm relative the trunk with a traditional exoskeleton it becomes hard to transfer load from the arm to the torso because the complexity of the motions of the shoulder joint. The shoulder not only have a ball joint between the scapula and humerus. The scapula itself is loosely connected to the ribs and the only rigid connection to the rest of the skeletal system is the collar bone. Therefore, to handle these motions, exoskeletons which allows shoulder movements usually have a gimbal at around the ball joint, but also often linkages to make the gimbal joint movable. Having misalignments of the joints of the exoskeleton to the body can cause pain and even lead to injury.

An external apparatus to assist arm lifting, in accordance with the preamble of independent claim 1, is known from US 4,559,932.

Summary of the invention

According to the invention an arm lifting support device is provided comprising a torso support and at least one artificial tendon for vertical lifting running at least from a pulling point provided at the torso support, over a shoulder of the user and connected to an arm attachment means for attachment of the at least one artificial tendon at an arm of a user in use. The pulling point is provided in a level above the collar bones of the user in use. A redirecting mean is positioned substantially vertically above the arm/shoulder joint of the user in use. In such a way it is possible to lift the arm into a level over the major socket/ball joint of the shoulder. It makes it also possible for the scapula to move in a natural way. Preferably, the pulling point is provided adjacent a neck portion of the torso support. Most preferred the pulling point is provided in a stiff neck portion. Thus the pulling point will not be dragged towards the arm to be lifted but stays in the right position

According to an embodiment of the invention a pulling force is applied to the artificial tendon, by a pulling means arranged along the artificial tendon or being comprised in the artificial tendon. Preferably, the arm lifting support device is arranged so that it does not limit the motion in the horizontal plane of the user's upper arm.

According to an embodiment of the invention the torso support further comprises a shoulder portion having at least two separate but connected shoulder parts. Preferably the at least two shoulder parts are partially overlapping. It is also possible to have three shoulder parts. Preferably an artificial tendon redirecting means is arranged at one of the shoulder parts.

According to an embodiment of the invention a control system is provided comprising a control means arranged to apply a desired pulling force in said at least one artificial tendon in a feedback loop, which pulling force is dependent upon a user posture measured using at least one posture sensor comprised in the control system. Preferably the said posture sensor is arranged to measure a torso and/or upper arm posture of the user.

According to an embodiment of the invention the control system is arranged to detect a current actual pulling force in said at least one artificial tendon, and wherein a feedback control loop is arranged to achieve said desired pulling force based upon said detected current actual force.

According to an embodiment of the invention the control system comprises a force regulator, arranged to achieve said desired pulling force in an internal feedback loop in said force regulator, based upon said sensed current actual force.

According to an embodiment of the invention the desired pulling force has been determined, for a number of different user postures, as a particular relative proportion of the force required to hold the user's arm still. Preferably, the control system is further arranged so that, for a given detected current actual non-zero force, the desired pulling force is stronger the closer to the horizontal the longitudinal axis of the user's upper arm is, as read by said posture sensor. The desired pulling force is substantially zero when the current orientation of the user's upper arm, as read by said posture sensor, is vertical or substantially vertical.

According to an embodiment of the invention the support device is arranged not to read any other postures or accelerations than using said at least one posture sensor.

According to an embodiment of the invention said at least one artificial tendon is the only force-mediating part of the control system between said points.

According to an embodiment of the invention the control system further comprises an elbow angle sensor, and that the control system is arranged not to apply any force to the artificial tendon in case the elbow angle sensor currently detects an unbent elbow. Preferably, none of said sensors is arranged to be in direct contact to the skin of the user. Each one of said sensors is fixedly attached to a main structure of the arm lifting support device itself.

According to an embodiment of the invention there is only one artificial tendon for vertical lifting. Preferably, a slidable attachment of the artificial tendon is provided wherein the arm attachment means is provided with means for a slidable attachment point of the artificial tendon at the arm attachment means, so that the attachment point can slide across a continuous path between a first and a second point at the user's arm. Preferably, the slidability is provided along an inside of an elbow joint, i.e. at the side having the cubital fossa, along which the attachment point can slide. A wire may be provided along the inside of the elbow joint and the slidable attachment of the artificial tendon is a loop attachment around the wire.

According to an embodiment of the invention the arm attachment means comprises two portions, an upper portion provided for attachment around an upper arm of a user when the arm lifting support device is in use and a lower portion provided for attachment at the lower arm of a user. The upper and lower portions may be connected with some sort of joint. Preferably, the arm attachment means embraces an elbow of a user in use.

Short description of attached drawing

The present invention will now be described in more detail by means of embodiments under referral to the attached drawing, in which:

Fig. 1 shows an embodiment of an arm lifting support device having a torso support.

Fig. 2 shows an embodiment of an artificial tendon running between a torso support and an arm attachment means.

Fig. 3 shows a front view of an embodiment of the slidability provided across an arm of a user when the arm lifting support device is in use.

Fig. 4 shows another embodiment of the slidability provided across an arm of a user in use.

Fig. 5 shows an embodiment of a shoulder portion of a torso support having an artificial tendon redirection means at the torso support.

Fig. 6 shows an embodiment of a neck portion of the torso support.

Fig. 7 shows an embodiment of an arm attachment means.

Fig. 8 shows an embodiment of an arm lifting support device from the side when worn by a user who lifts his arm in front of him.

Fig. 9a, b and c shows an embodiment of a sensor arranged for sensing a position of an arm of a user, the arm shown in different positions.

Fig. 10 shows an embodiment of a feedback loop.

Fig. 11 shows an alternative positioning of the pulling point.

Detailed description of embodiments of the present invention

The present invention will be described in detail by means of embodiments. This description does not limit the scope of the invention. The scope is depicted by the following claims.

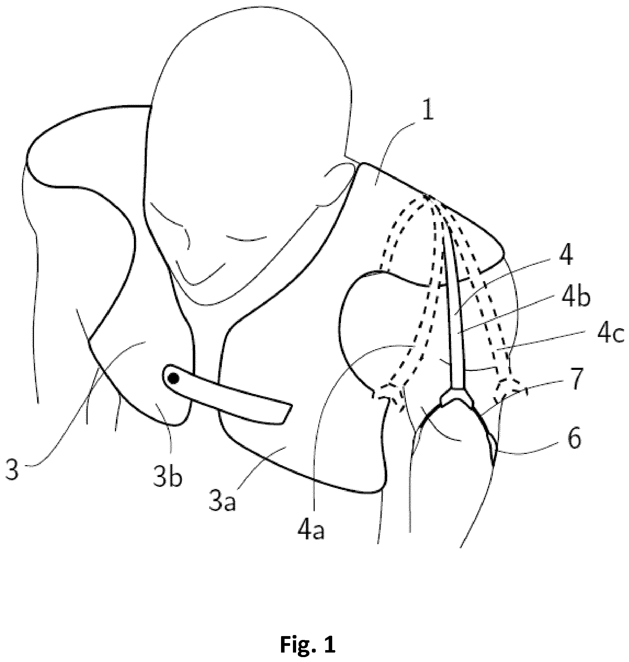

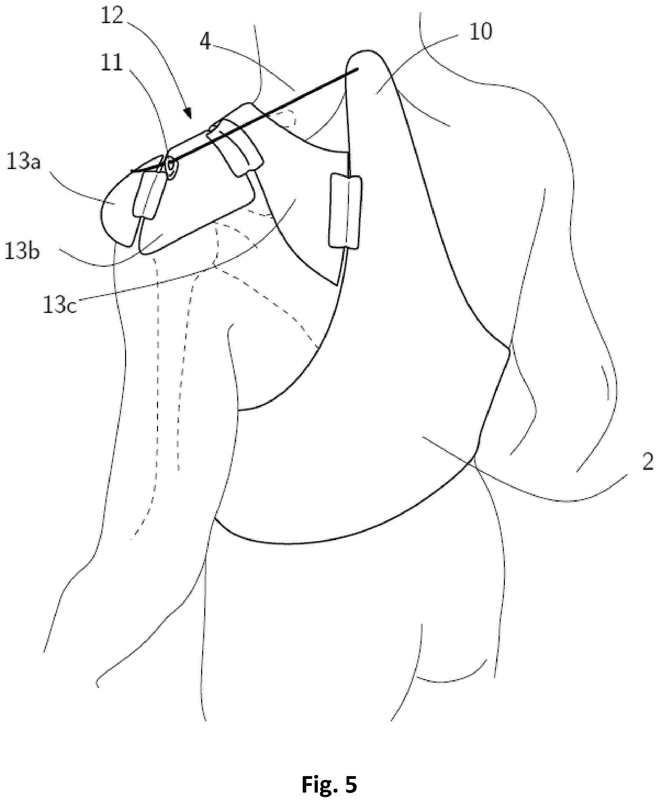

In Fig. 1 an arm lifting support device is shown having a torso support 1, for example made up by a back section 2 (see Fig. 5), a front section 3 comprising a first part 3a and a second part 3b. Both parts may be interconnected with the back section 2 and detachably connected to each other at the front. It is of course possible having another configuration of the torso support 1.

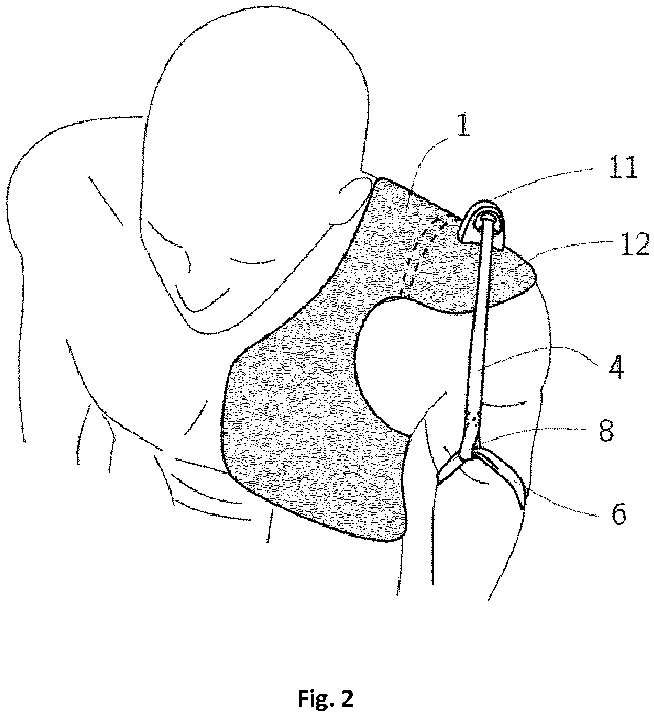

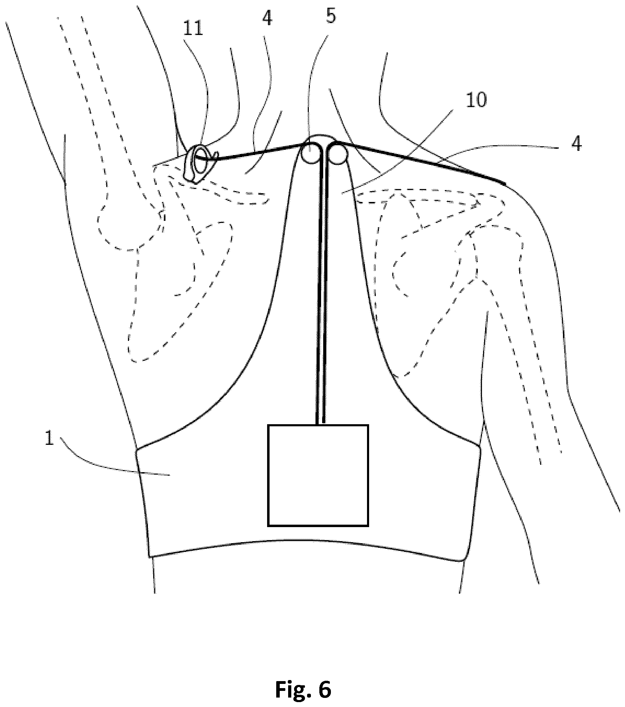

At least one artificial tendon 4 runs at least from a pulling point 5, see Fig. 6, provided at the torso support 1 and is connected at an arm attachment means 6, see Fig. 1 and 2. The artificial tendon 4 runs over a shoulder part 12 of the torso support 1 between the pulling point 5 and the arm attachment means 6. The shoulder part 12 may be a portion of the torso support 1 having a bending line for facilitate bending of the shoulder part upwards, as shown in Figs. 1 and 2, or separate but connected parts as shown in Fig. 5. The arm attachment means 6 is positioned at an arm of a user when the arm lifting support device is in use so that the artificial tendon 4 will be attached to the arm of the user. It is possible to have more than one artificial tendon, for example attached at either side of the arm attachment means 6 or for side to side movement of the arm of the user in a plane.

However, it is an advantage if only one artificial tendon 4 is used, at least for the lifting of the arm of the user. In order to avoid bringing unwanted momentum to the arm of the user, rotation around its length axis, even if only one artificial tendon 4 is used, the present invention provides a slidable attachment of the artificial tendon 4 at the arm attachment means 6.

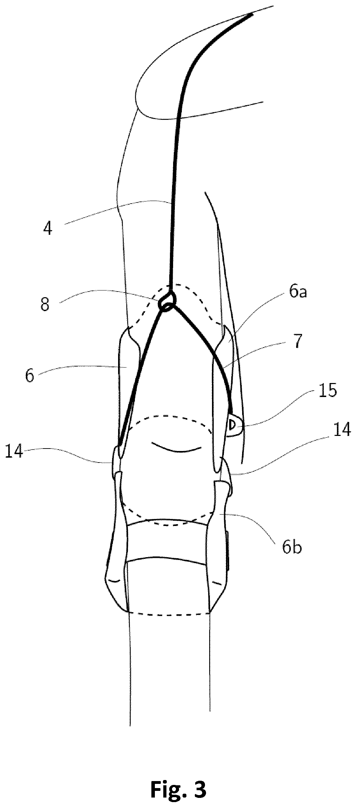

The artificial tendon 4 is attached at an attachment point to the attachment means so that the attachment point can slide across a continuous path between a first and a second point at the user's arm. The slidability is provided across the arm of the user, from side to side or all around the circumference of the user's arm, along which the attachment point can slide. The slidability is preferably provided adjacent the elbow joint of the user's arm during use. At least, the slidability is provided at the inside of the elbow joint. For example, it may be a wire 7 running from side to side of the elbow joint, preferably on the inside thereof, as can be seen in Fig. 3. For example, the artificial tendon 4 may connect with the wire 7 by means of a loop 8. This type of solution may also be seen in Figs. 1 and 2.

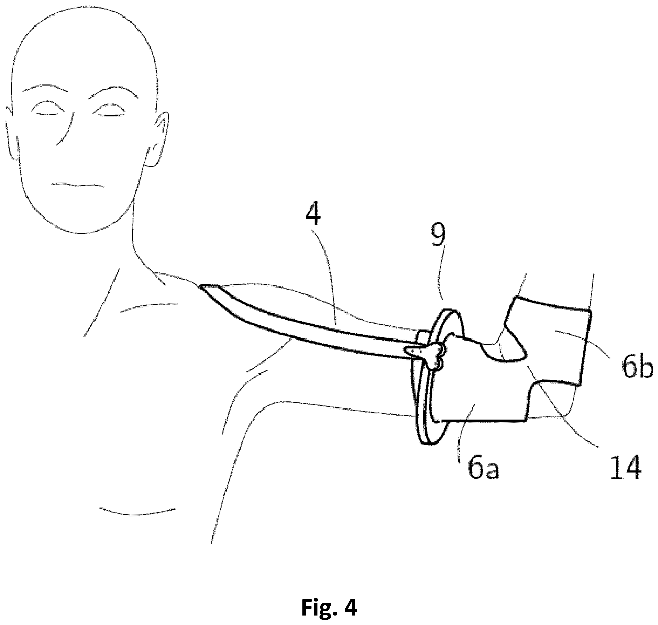

If the slidability is to be provided all around the arm of the user it may be a rail 9 encircling the user's arm when provided at the arm in use. See Fig. 4. The connection between the artificial tendon 4 and the rail 9 may for example be a form fit type of connection where the connection may travel along the rail 9 but without detaching from it.

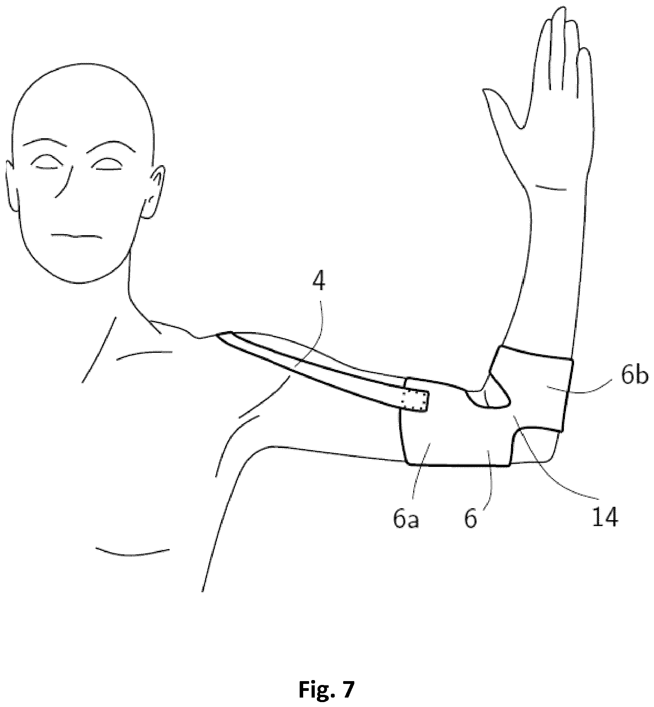

In Fig. 7, an embodiment of an arm attachment means 6 is shown. The arm attachment means 6 comprises two portions, an upper portion 6a provided for attachment around an upper arm of a user in use and a lower portion 6b provided for attachment at the lower arm of a user.

An adjustment device 15 (Fig. 3) is attached to the upper portion 6a of the arm attachment means 6. With the adjustment device 15 the length of the wire 7 can be adjusted manually or automatically so the wire 7 better fits the user.

The two portions 6a, b are preferably interconnected. The interconnection 14 could be a joint or a soft portion in order not to delimit the possibility to flex the arm for the user. Preferably there are two interconnections 14, one on each side. In order to minimize the risk of sliding of the arm attachment means 6 upwards along the arm of the user it is preferably arranged embracing the elbow of the user's arm in use. The slidability function 7, 9 may be attached close to the interconnections 14.

In one embodiment, the interconnection is made of two strings, wires or the like that connects two points of each portion and keeps their relative distance constant.

In Fig. 6 the pulling point 5 for the artificial tendon 4 is positioned in a level above the collar bones of a user in use. It is preferably arranged in the vicinity of the neck portion 10 of the torso support 1. The neck portion 10 is preferably stiff in at least the lateral direction, which is advantageous for the force distribution in the torso support 1 and delimits the risk of displacement of the pulling point 5, too. If the torso support 1 is of the kind making it possible for the user to get dressed on her or his own, the stiff neck portion will be of help in the dressing movements.

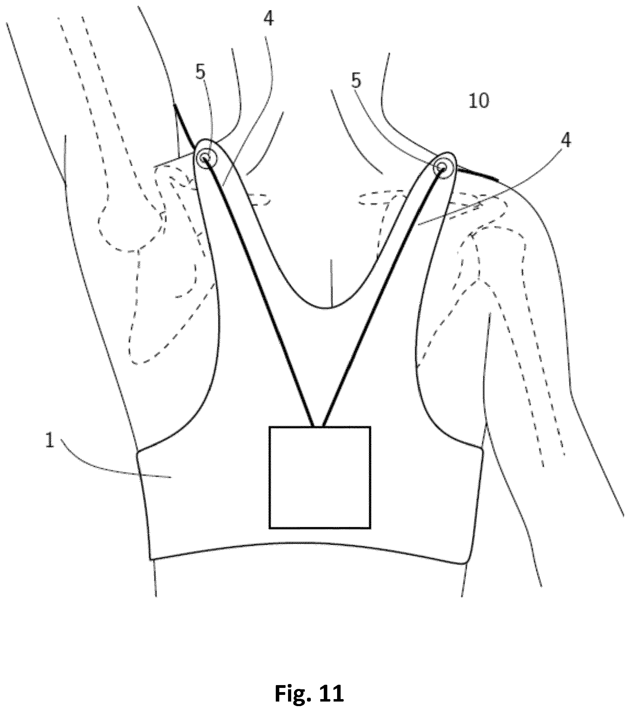

An alternative position of the pulling point 5 is illustrated in Fig. 11, where it is positioned behind the shoulder, somewhere from in between the shoulder and neck. This alternative can be combined with the tendon 4 redirecting means 11 or not.

As can be seen in Fig. 5 an artificial tendon 4 redirecting means 11 is provided at the torso support 1. Preferably the redirecting means 11 is in use positioned vertically above the major ball and socket joint (glenohumeral joint) at the shoulder/arm of the user. The redirecting means 11 may be arranged at a shoulder portion 12 of the torso support 1.

According to the shown embodiment the shoulder portion comprises two separate but connected shoulder parts 13a and 13b. It is also conceivable to arrange three or more shoulder parts 13 in the shoulder portion 12. The shoulder parts 13 a, b may be partially overlapping or flexibly arranged side by side. By having these parts separate but connected makes the torso support able to better follow the movement of the body, especially during high lifting of the arm of the user, without hindering or counteract the movement of the arm of the user. The redirecting means 11 is provided at one of the shoulder parts 13, in the shown case the inner part 13b as seen from the neck portion 10.

The shoulder part may have a smooth upper side such that the artificial tendon 4 can slide transverse its axis when the arm is doing lateral movements. Fig. 1 illustrates this with different orientations of the artificial tendon 4. When the arm of the user is in a front position the artificial tendon 4 is in a front position 4a. When the arm of the user is in a neutral position the artificial tendon 4 is in a neutral position 4b and when the arm is in a lateral position (sideway position) the artificial tendon 4 is in a lateral position 4c.

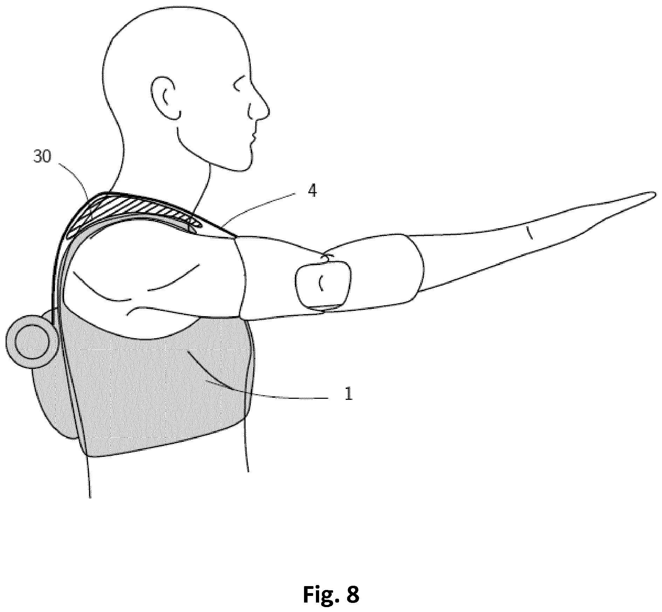

The shoulder part may preferably be padded 30 as illustrated in Fig 8 so that the distance upwards from the shoulder major ball and socket joint increases. Thus, the required forces on the artificial tendon 4 will be reduced.

According to a preferred embodiment of the present invention, a control system 20 is provided comprising a control means 21, arranged to impart a desired predetermined or calculated pulling force to the artificial tendon 4.

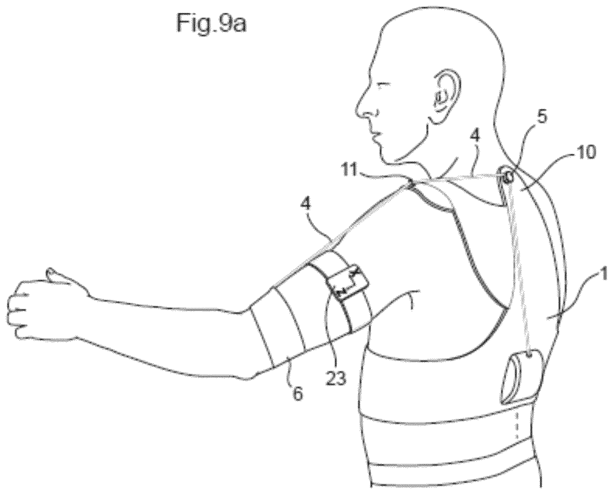

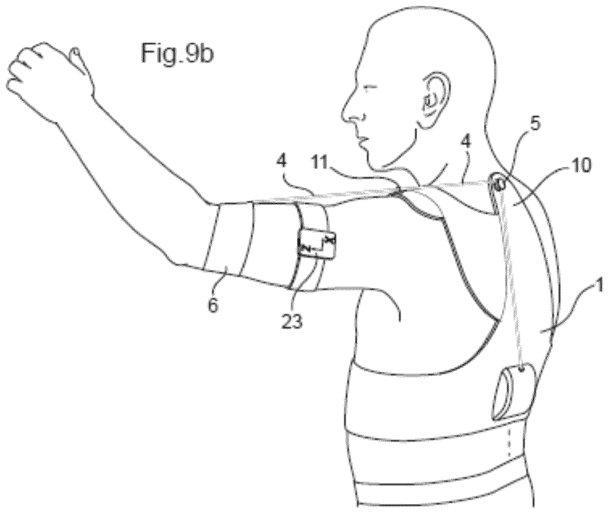

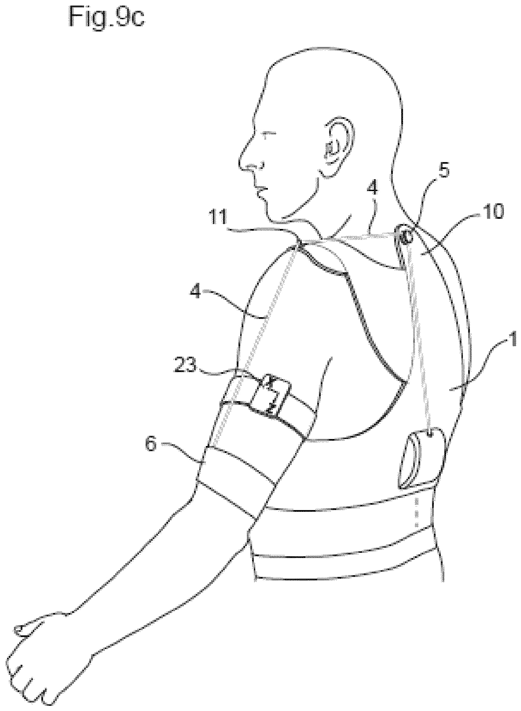

It is preferred that the control system 20 further comprises one or several posture sensors 23, arranged to detect a current orientation of at least one of the user's torso and the user's upper arm, in relation to the horizontal plane. Such a sensor 23 may, for instance, be arranged on the said torso or anywhere along the upper arm of the user, such as at or near the elbow or as depicted in figure 9a-c, along the upper arm. The posture sensor 23 may comprise an accelerometer and/or a gyro, and may for instance be an as such conventional IMU unit. It may also comprise an angle sensor (a goniometer), measuring the relative angle between different body parts. The posture sensor 23 is further arranged to provide torso and/or upper arm orientation measurement values of the said type to the control means 21, such as using a wire or a wireless communication channel.

Then, the control means 21 is preferably arranged to impart said desired pulling force so that it depends upon the said torso and/or arm orientation as read by said one or several posture sensors 23.

It is possible that the desired force is applied using an entirely passive system, such as using entirely mechanical spring means. However, according to a further preferred embodiment, the control system 20 also comprises an actual force sensor 22 used in a force control feedback loop of the control system 20. It is realized that such an actual force sensor 22 may be arranged as an integral part of the control means 21, or in any other suitable way.

In this feedback loop case, the actual force sensor 22 is arranged to detect a current actual pulling force in the artificial tendon 4. In case there are several artificial tendons 4, it is preferred that the actual force sensor 22 is arranged to estimate a total actual force acting on the arm based upon force values measured on several such artificial tendons. One simple example of how this estimation can be done is to detect a maximum actual current force in the artificial tendons in question. The actual force sensor 22 is further arranged to feed the said detected actual force value to the control means 21, which in turn is arranged to control the pulling force along said at least one artificial tendon 4 to said desired force value.

The pulling force may be controlled using a particular regulation metric, such as a voltage, a current, a power, a pneumatic or hydraulic pressure, or the like, which in turn is set by the control device and which affects the applied pulling force by acting on the tendon or tendons 4 using a suitable actuation mechanism, such as an electric motor or a pneumatic system.

As described above, several artificial tendons 4 may be used in parallel, and controlled individually or in unison. In the following, however, the description assumes that there is only one artificial tendon 4. It is realized that all that is said regarding the control system 20 in relation to such single artificial tendon 4 is correspondingly applicable also to the case of several tendons.

The desired force may be achieved using a suitable, conventional pulling means, such as a roller means arranged to roll the artificial tendon 4 onto a roll, thereby shortening/lengthening the tendon 4. It may also be a driven using a screw drive, in turn powered electrically, pneumatically or hydraulically; a pneumatic artificial muscle ("McKibben muscle"); an electroactive polymer fibre; or the like. Preferably, when so is possible (such as in the two latter examples), the force-imparting means forms a part of the tendon itself.

The magnitude of the regulation metric is in general preferably dependent upon the measured actual tendon 4 force. More particularly, the control means 21 is preferably arranged to achieve said desired force according to a feedback control loop, based upon said detected current actual force. The feedback control loop may be an algorithm implemented by the control system in hardware, software or a combination of the two.

Such a feedback control loop can preferably be designed so that the desired pulling force compensates for the force of gravity acting on the user's arm in a way which is perceived as "natural" in the sense that the normal resistance felt by the user's arm due to gravity is still present but reduced, so that a movement intended and initiated by the user will be performed in a natural way but with less required user strength. To accomplish this, it is preferred that the control of the pulling force is performed dynamically in the sense that it acts on the moving artificial tendon 4. In other words, as the tendon 4 is shortened or lengthened as a result of the user's arm being raised or lowered, the desired pulling force is applied to the shortened or lengthened artificial tendon 4, for instance by rotating the above described roll while the tendon 4 is rolled on or off from the roll in question.

Preferably, the desired pulling force is not strong enough to lift the arm, even not strong enough to keep the arm in a stable position when raised above a vertical position. In particular, the control means 21 is not arranged to initiate any movement of the arm, in other words the feedback loop is preferably entirely reactive in its design.

Preferably, the desired pulling force does not depend upon whether the arm is currently being raised or lowered. However, according to one alternatively preferred embodiment, a differently designed feedback control loop may indeed be implemented for upwards arm movements as compared to downwards arm movements. This may, for instance, be useful if used as a load-decreasing device for repetitive assembly work or the like, or if a particular rehabilitation scheme is to be implemented for particular patient groups. The current movement direction may preferably be dynamically detected, such as by an arm posture sensor of the above described type.

It is preferred that the control system 20 comprises a force regulator, arranged to achieve the said desired force in an internal feedback loop in said force regulator, based upon said sensed current actual force. In this case, it is the force regulator which controls the value of the said regulation metric so as to achieve the desired force. Such a force regulator may have a built-in force sensor for detecting the current actual force, or be connected to an external force sensor, such as arranged on the tendon 4, at the pulling point or on the user's arm.

Such a design of the control system 20 makes it possible for one single actuator, in the form of the control means 21, to assist all arm and shoulder movements of the user. This is especially true in a support device of the present type, providing vertical arm lifting assistance but not affecting horizontal arm movements per se. Since the actual force sensor 22 can be arranged at or as a part of the control means 21, the whole control system 20 can be arranged as one single device, for instance at the above described pulling point 5 or, via a tendon 4 redirection means at the pulling point 5, elsewhere, such as in a control package on the user's back.

According to a preferred embodiment, the control system 20 comprises an adjustment means 24, via which a relative strength of the desired tendon 4 pulling force can be adjusted by the user. Such an adjustment will preferably result in the desired force being altered for each sensed arm and/or torso posture, preferably in a linear manner across the whole observed such posture interval. Using such an adjustment means 24, the user can adjust the overall level of assistance provided by the control system 20 in a simple manner, without affecting the general behaviour of the control system 20.

Furthermore, it is preferred that the feedback loop is arranged so that the desired pulling force is stronger the closer to the horizontal the longitudinal axis of the user's upper arm is, as read by said posture sensor(s) 23. This means that, moving from a vertically downward arm position to a horizontal arm position, the pulling force will be larger. Above the horizontal, the pulling force may again be less than at the horizontal. For instance, the desired force may be varied so that it is at a maximum when the upper arm is in said horizontal position.

As the current orientation of the user's upper arm is vertical, or substantially vertical, as read by said posture sensor, the desired pulling force is preferably zero or substantially zero.

In a corresponding manner, the desired pulling force may be varied as a function of an orientation of the user's torso, as read by a corresponding posture sensor 23. In the latter case, it is preferred that the desired force is varied as a function of such a read posture so that more upright torso positions yield a greater assisting desired force. This may or may not be combined with the above-described arm position-dependent variation.

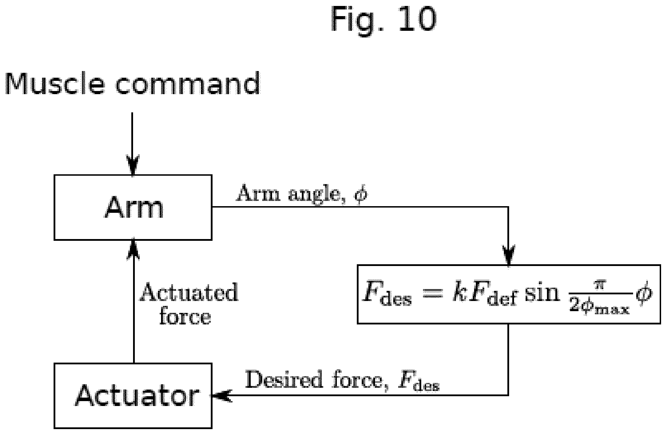

Figure 10 illustrates an example of a feedback mechanism according to the present invention, arranged to read the upper arm posture angle $\phi$ and to apply a desired pulling force $F_{des}$, with the aim of maintaining said desired tendon pulling force in dependence upon said read arm posture angle. The force regulator internally measures the actual force, and regulates the pulling force in dependence thereupon. The general level of assistance can be controlled by the user, by selecting a suitable value for the constant k, for instance using an adjustment knob of the above mentioned type. In the example, the desired pulling force is the scaled sine function of the measured current arm posture angle. It is realized that different and/or more complex desired force functions may be applied, depending on the particular needs:

$$F_{des} = kF \ sin\left(\frac{\pi}{2\phi_{max}}\phi\right),$$

wherein

k is said constant controlling the general level of assistance;

$F_{def}$ is a predetermined or adjustable default desired pulling force;

$\phi_{max}$ is a predetermined or adjustable (see below) arm posture angle (such as horizontally oriented arm) at which the desired pulling force is to be at its peak; and

$\phi$ is the measured arm posture angle.

In general, it is preferred that the internal feedback loop is designed to operate in substantially the same principal corresponding way across the complete interval of detected upper arm orientations. In particular, it is preferred that the detected upper arm orientation only affects the feedback loop by one or several constant or variable (across upper-arm posture values) compensating factors or variables to the desired tendon pulling force value, for instance so that the compensating factor is applied to the default pulling force $F_{def}$ or as an argument to an angle-dependent function used to calculate $F_{des}$, before using the desired pulling force in the above-described internal feedback loop. This makes it possible to separate the posture sensor 23 based functionality from the rest of the control system 20, so that the posture sensor 23 can be switched on or off while maintaining the internal feedback functionality. Also, the posture sensor 23 may be post-installed onto an existing control system 20. The corresponding is also true regarding a torso posture-based feedback loop, as described above.

In figure 10, there are two such compensating factors - $k$ and $\frac{\pi}{2\phi_{max}}$.

In general, the desired compensating force may be calculated as or based upon an at least section-wise continuous function, such as a sine function, of the detected arm and/or torso posture. It is preferred that a separate adjustment means 25, such as a knob, is provided for adjusting at what upper-arm orientation value $\phi_{max}$ the maximum desired pulling force is to be applied, such as by the separate adjustment means.

According to one preferred embodiment, the posture-dependent desired force function is determined empirically in an initial configuration step, performed using a suitable software function which may for instance be executed on a hardware processor in the control system 20. During such configuration, the posture of the arm and/or the torso of the user is varied, such as by the actuator driving or aiding the user to move the arm to a set of preferably predetermined orientations, and sensed by one or several corresponding posture sensors, as described above. At the time for such sensing, it is preferred that the weight of the arm, preferably unaided by the user's muscle force, is imparted to the sensor. At the same time, the current actual tendon 4 force may be read, and the resulting dataset, comprising measured actual tendon 4 forces for a number of different known user postures, is used to calculate the said posture-dependent desired tendon force function for use during operation of the control system 20.

For instance, the user can be instructed to move his or her arm and/or torso in a predetermined motion pattern, which posture sequence is then detected together with corresponding actual tendon 4 force measurements for each detected posture, and stored in a digital memory by the control system 20. In one embodiment, the control system 20 is arranged to control an applied pulling force, in a way corresponding to the one described above, so as to barely or precisely offer the required assistance to the user so as to allow the user to maintain each of a series of predetermined postures, while recording the detected corresponding posture(s) and the actual tendon force.

During the configuration, the user may be instructed to perform a series of postures while exerting maximum force. This will automatically create a benchmark for the force the user is able to exert across a range of different torso and/or upper arm postures, based upon which the said desired force function can be automatically determined by the control system 20 software. Thereafter, the user may control the actual force assistance during use by adjusting the said adjustment knob or knobs described above, depending on current fatigue level or type of activity.

As a result of such configuration, or based upon predetermined or manual parameter settings, it is preferred that the desired tendon force in the above discussed feedback control loop is determined, for a number of different user postures, as a particular relative proportion of the force required to hold the user's arm still, with or without using any muscle power, at the posture in question. This proportion may then be adjusted using the adjustment knob or knobs as described herein.

During a configuration step of the above-described type, it is also preferred that a horizontal upper arm posture is automatically determined, for instance by the user being instructed to position the arm into such a horizontal posture and allowing the control system 20 to then detect the actual posture sensor data when in this posture, and to use this data as a horizontal posture reference in the above described feedback algorithms.

According to an additional preferred embodiment, the control system 20 is configurable, for instance during such a configuration step, to allow for a stronger assistance in particular predetermined posture intervals, such as at postures at which the user, in a current activity such as when shopping or performing a particular repetitive assembly task, must exert more force. Such intervals, and such extra assistance force, is then implemented in said posture-dependent desired tendon force function, preferably as a software function in said control device.

Further preferably, the control system 20 further comprises an elbow angle sensor 26, which is arranged to detect a current flexing angle of the user's elbow, and which is connected to the control means 21, for delivering elbow angle measurement values, in a way which may be similar to the connection of the posture sensor 23. Preferably, in the case in which both a posture sensor 23 and an elbow angle sensor 26 are used, they may be arranged as one single integrated unit, using one single communication interface for communicating with the control means 21. In this case, it is preferred that such integrated unit is arranged at the elbow, such as on the arm attachment means 6, possibly as a part of the interconnection 14.

In case an elbow angle sensor 26 is used, the feedback control loop of the control system 20 is arranged so that no or substantially no pulling force is applied to the artificial tendon 4 in case the elbow angle sensor currently detects an unbent elbow. Apart from this function, the elbow angle sensor 26 detected value preferably has no effect on the applied pulling force. This will avoid the arm attachment means 6 being pulled directly inwards towards the user's shoulder in case the arm is completely straight, thereby bringing the arm attachment means 6 out of place. Hence, this embodiment is most preferred in case the pulling point 5 and possible tendon redirection means 11 are positioned so that the pulling force is applied along a line which is relatively near the ball-and-socket shoulder joint, preferably so that the tendon 4 runs substantially in parallel to the upper arm of the user. Preferably, an elbow flexing angle of about 5°, or even less, will result in the normal feedback control loop to be applied.

In addition to measuring the posture of the arm and/or the torso, as described above, similar posture sensors can also be used to measure other geometric posture aspects of the user's arm and with the aim of modifying the above-described desired force based upon such measurements. Examples comprise the position of the upper arm, in relation to the user's torso, in the horizontal plane; the rotating/twisting angle of the arm; and/or the flexing of the elbow. For instance, greater desired forces (and hence more powerful support to the user) can be used when the arm is located and moved in the median plane as compared to in the frontal plane; and a more flexed arm may require a smaller desired pulling force.

Similarly, the desired pulling force may by dynamically determined in reaction to a detected first- or higher order derivative of an arm posture angle, and/or of any other read posture. In particular, it is desired that faster ongoing posture changes, in particular arm movements, result in larger desired pulling forces, leading to a more naturally-feeling assistance due to normally occurring viscous friction effects in human arms.

Furthermore, the support device, and in particular the control system 20, is preferably arranged not to read any other postures or accelerations than using said posture sensor(s) 23 (in particular, preferably only an upper arm posture sensor in case no torso posture sensor is used) and, if applicable, said elbow angle sensor 26. This provides for a very simple yet versatile and useful system for providing user-initiated arm movement assistance to a user.

For similar reasons, it is preferred that said at least one artificial tendon 4 is the only force-mediating part of the control system 20 between the pulling point 5 and the arm attachment means 6.

According to a preferred embodiment, none of said sensors 23, 26 is arranged to be in direct contact to the skin of the user. Having a sensor arranged in direct contact with the skin can be perceived as uncomfortable by the user, and using the present support device such arrangement is not necessary. Instead, the sensors can be arranged on the main support device parts 6, 12, etc. In particular, it is preferred that the sensors 23, 26 are not arranged to read nervous system signals from the user of the like, but that they are arranged to read purely mechanical posture and angle data based upon their orientation in space, and possibly also based upon their orientation in relation to other parts of the support device.

In particular, each one of said sensors 23, 26 is preferably fixedly attached to a main structure of the arm lifting support device itself, in such a way so that it does not come into direct contact with the user's skin when worn. For instance, the sensors may be fastened on top of a layer of textile fabric.

Apart from the above described sensors, additional sensors may be used in some embodiments, for controlling the feedback loop but in particular to achieve specific temporary functionality based upon specific measured circumstances. For instance, an electromyographic (EMG) sensor may be used to temporarily providing an extra boost, such as when a user muscular effort is detected in relevant muscular tissue. This results in a more complex product, and is therefore not preferred in some embodiments in which simplicity is sought.

It is understood that the above described control system 20 may be implemented to assist arm movements of only one of the user's arms, or both. In the latter case, the feedback functionality may be identical or different, depending on the needs of the user. In one preferred embodiment, the above discussed configuration is performed independently for each of the user's two arms, and a respective resulting feedback loop is then applied independently to the movements and postures of each respective arm.

**Claims**

1. An arm lifting support device, comprising a torso support (1), at least one artificial tendon (4) for vertical lifting, running at least from a pulling point (5) provided at the torso support, over a shoulder of the user and connected to an arm attachment means (6) for attachment of the at least one artificial tendon at an arm of a user when the arm lifting support device is in use, **characterised in that** the pulling point is provided in a level above the collar bones of the user in use and a redirecting means (11) is provided vertically above the major ball and socket joint, which is the glenohumeral joint, of the user in use.

2. The arm lifting support device according to claim 1, wherein a pulling force is applied to the artificial tendon by a pulling means arranged along the artificial tendon or being comprised in the artificial tendon.

3. The arm lifting support device according to claim 2, wherein the pulling means is arranged to apply said pulling force at a pulling point arranged adjacent a neck portion (10) of the torso support.

4. The arm lifting support device according to claim 3 wherein the pulling point is provided in a stiff neck portion.

5. The arm lifting support device according to any one of the preceding claims, wherein the torso support comprises a at least one shoulder part (13) having a smooth upper side such that the artificial tendon can slide transverse its axis when a user is doing lateral movements.

6. The arm lifting support device according to any one of the preceding claims, wherein a control system (20) is provided comprising a control means (21) arranged to apply a desired pulling force in said at least one artificial tendon in a feedback loop, which pulling force is dependent upon a user posture measured using at least one posture sensor (23) comprised in the control system.

7. The arm lifting support device according to claim 6, wherein the said posture sensor is arranged to measure a torso and/or upper arm posture of the user.

8. The arm lifting support device according to claim 6 or 7, wherein the control system is arranged to detect a current actual pulling force in said at least one artificial tendon, and wherein a feedback control loop is arranged to achieve said desired pulling force based upon said detected current actual force.

9. The arm lifting support device according to claim 8, wherein the control system comprises a force regulator, arranged to achieve said desired pulling force in an internal feedback loop in said force regulator, based upon said sensed current actual force.

10. The arm lifting support device according to any one of claims 6-9, wherein the control system is further arranged so that, for a given detected current actual non-zero force, the desired pulling force is set stronger the closer to the horizontal the longitudinal axis of the user's upper arm is, as read by said posture sensor.

11. The arm lifting support device according to any one of claims 6-10, wherein said at least one artificial tendon is the only force-mediating part of the control system between said pulling point and said arm attachment means.

12. The arm lifting support device according to any one of claims 6-11, wherein the control system further comprises an elbow angle sensor (26), and that the control system is arranged not to apply any force to the artificial tendon in case the elbow angle sensor currently detects an unbent elbow.

**Patentansprüche**

1. Eine Armhebeunterstützungsvorrichtung, umfassend eine Rumpfstütze (1), mindestens eine künstliche Sehne (4) zum vertikalen Heben, wobei die Sehne mindestens von einem an der Rumpfstütze vorgesehenen Zugpunkt (5) über eine Schulter des Benutzers verläuft und mit einem Armbefestigungsmittel zum Befestigen der mindestens einen künstlichen Sehne an einem Arm eines Benutzers verbunden ist, wenn die Armhebeunterstützungsvorrichtung in Gebrauch ist, **dadurch gekennzeichnet, dass** der Zugpunkt im Gebrauch in einer Ebene über dem Schlüsselbein des Benutzers vorgesehen ist und eine Umlenkeinrichtung (11) im Gebrauch vertikal über dem großen Kugelgelenk, dem Glenohumeralgelenk, des Benutzers vorgesehen ist.

2. Die Armhebeunterstützungsvorrichtung nach Anspruch 1, wobei eine Zugkraft auf die künstliche Sehne durch ein Zugmittel, das entlang der künstlichen Sehne angeordnet ist oder in der künstlichen Sehne enthalten ist, ausgeübt wird.

3. Die Armhebeunterstützungsvorrichtung nach Anspruch 2, wobei das Zugmittel so angeordnet ist, dass es die Zugkraft an einem Zugpunkt ausübt, der neben einem Halsabschnitt (10) der Rumpfstütze angeordnet ist.

4. Die Armhebeunterstützungsvorrichtung nach Anspruch 3, wobei der Zugpunkt in einem steifen Halsabschnitt vorgesehen ist.

5. Die Armhebeunterstützungsvorrichtung nach einem der vorstehenden Ansprüche, wobei die Rumpfstütze mindestens ein Schulterteil (13) mit einer glatten Oberseite umfasst, so dass die künstliche Sehne quer zu ihrer Achse gleiten kann, wenn ein Benutzer seitliche Bewegungen ausführt.

6. Die Armhebeunterstützungsvorrichtung nach einem der vorstehenden Ansprüche, wobei ein Steuersystem (20) vorgesehen ist, das ein Steuermittel (21) umfasst, das so angeordnet ist, dass es in einer Rückkopplungsschleife eine gewünschte Zugkraft auf die mindestens einen künstlichen Sehne ausübt, wobei die Zugkraft von einer Benutzerhaltung, die unter Verwendung mindestens eines in dem Steuersystem enthaltenen Haltungssensors (23) gemessen wird, abhängig ist.

7. Die Armhebeunterstützungsvorrichtung nach Anspruch 6, wobei der genannte Haltungssensor so angeordnet ist, dass er eine Rumpf- und/oder Oberarmposition des Benutzers misst.

8. Die Armhebeunterstützungsvorrichtung nach Anspruch 6 oder 7, wobei das Steuersystem vorgesehen ist, um eine aktuelle Ist-Zugkraft in der mindestens einen künstlichen Sehne zu erfassen, und wobei eine Rückkopplungsregelschleife vorgesehen ist, um die gewünschte Zugkraft auf der Grundlage der erfassten aktuellen Ist-Kraft zu erreichen.

9. Die Armhebeunterstützungsvorrichtung nach Anspruch 8, wobei das Steuersystem einen Kraftregler umfasst, der vorgesehen ist, um in einer internen Rückkopplungsschleife die gewünschte Zugkraft in dem Kraftregler auf der Grundlage der erfassten aktuellen Ist-Kraft zu erreichen.

10. Die Armhebeunterstützungsvorrichtung nach einem der Ansprüche 6-9, wobei das Steuersystem ferner vorgesehen ist, um für eine gegebene erfasste aktuelle Ist-Kraft, die nicht Null ist, die gewünschte Zugkraft umso stärker einzustellen, je näher die Längsachse des Oberarms des Benutzers an der Horizontalen liegt, was von dem Haltungssensor ausgelesen wird.

11. Die Armhebeunterstützungsvorrichtung nach einem der Ansprüche 6-10, wobei die mindestens eine künstliche Sehne der einzige kraftvermittelnde Teil des Steuersystems zwischen dem Zugpunkt und dem Armbefestigungsmittel ist.

12. Die Armhebeunterstützungsvorrichtung nach einem der Ansprüche 6-11, wobei das Steuersystem ferner einen Ellbogenwinkelsensor (26) umfasst und das Steuersystem vorgesehen ist, um keine Kraft auf die künstliche Sehne auszuüben, falls der Ellbogenwinkelsensor einen aktuell nicht gebogenen Ellbogen erkennt.

**Revendications**

1. Dispositif de support de levage de bras comprenant un support de torse (1), au moins un tendon artificiel (4) pour le levage vertical, s'étendant au moins à partir d'un point de traction (5) prévu au niveau du support de torse, sur une épaule de l'utilisateur et raccordé à un moyen de fixation de bras (6) pour la fixation du au moins un tendon artificiel au niveau d'un bras d'un utilisateur lorsque le dispositif de support de levage de bras est utilisé, **caractérisé en ce que** : le point de traction est prévu dans un niveau au-dessus des clavicules de l'utilisateur, à l'usage, et un moyen de redirection (11) est prévu verticalement au-dessus de l'articulation sphéroïde principale, qui est l'articulation glénohumérale, de l'utilisateur, à l'usage.

**2.** Dispositif de support de levage de bras selon la revendication 1, dans lequel une force de traction est appliquée sur le tendon artificiel, par un moyen de traction agencé le long du tendon artificiel ou étant compris dans le tendon artificiel.

**3.** Dispositif de support de levage de bras selon la revendication 2, dans lequel le moyen de traction est agencé pour appliquer ladite force de traction au niveau d'un point de traction agencé de manière adjacente à une partie de cou (10) du support de torse.

**4.** Dispositif de support de levage de bras selon la revendication 3, dans lequel le point de traction est prévu dans une partie de cou rigide.

**5.** Dispositif de support de levage de bras selon l'une quelconque des revendications précédentes, dans lequel le support de torse comprend au moins une partie d'épaule (13) ayant un côté supérieur lisse de sorte que le tendon artificiel peut coulisser transversalement par rapport à son axe lorsqu'un utilisateur réalise des mouvements latéraux.

**6.** Dispositif de support de levage de bras selon l'une quelconque des revendications précédentes, dans lequel on prévoit un système de commande (20) comprenant un moyen de commande (21) agencé pour appliquer une force de traction souhaitée dans ledit au moins un tendon artificiel dans une boucle de rétroaction, laquelle force de traction dépend de la posture de l'utilisateur mesurée à l'aide d'au moins un capteur de posture (23) compris dans le système de commande.

**7.** Dispositif de support de levage de bras selon la revendication 6, dans lequel ledit capteur de posture est agencé pour mesurer une posture de torse et/ou de bras de l'utilisateur.

**8.** Dispositif de support de levage de bras selon la revendication 6 ou 7, dans lequel le système de commande est agencé pour détecter une véritable force de traction courante dans ledit au moins un tendon artificiel, et dans lequel une boucle de commande de rétroaction est agencée pour obtenir ladite force de traction souhaitée sur la base de ladite véritable force courante détectée.

**9.** Dispositif de support de levage de bras selon la revendication 8, dans lequel le système de commande comprend un régulateur de force, agencé pour obtenir ladite force de traction souhaitée dans une boucle de rétroaction interne dudit régulateur de force, sur la base de ladite véritable force courante détectée.

**10.** Dispositif de support de levage de bras selon l'une quelconque des revendications 6 à 9, dans lequel le système de commande est en outre agencé de sorte que, pour une force non nulle véritable courante détectée donnée, la force de traction souhaitée est plus puissante, plus l'axe longitudinal du bras de l'utilisateur est proche de l'horizontale, comme lu par ledit capteur de posture.

**11.** Dispositif de support de levage de bras selon l'une quelconque des revendications 6 à 10, dans lequel ledit au moins un tendon artificiel est la seule partie intermédiaire de force du système de commande entre ledit point de traction et ledit moyen de fixation de bras.

**12.** Dispositif de support de levage de bras selon l'une quelconque des revendications 6 à 11, dans lequel le système de commande comprend en outre un capteur d'angle de coude (26), et en ce que le système de commande est agencé pour ne pas appliquer de force sur le tendon artificiel au cas où le capteur d'angle de coude détecte un coude non plié.

**Fig. 1**

**Fig. 2**

**Fig. 3**

**Fig. 4**

**Fig. 5**

**Fig. 6**

**Fig. 7**

**Fig. 8**

Fig.9a

Fig.9b

Fig.9c

Fig. 10

Muscle command

Arm

Arm angle, $\phi$

Actuated
force

$$F_{\mathrm{des}} = kF_{\mathrm{def}} \sin \frac{\pi}{2\phi_{\mathrm{max}}}\phi$$

Actuator

Desired force, $F_{\mathrm{des}}$

**Fig. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 4559932 A